(19) **Europäisches Patentamt / European Patent Office / Office européen des brevets**

(11) **EP 2 527 815 B1**

(12) # EUROPEAN PATENT SPECIFICATION

(45) Date of publication and mention
of the grant of the patent:
**14.05.2014 Bulletin 2014/20**

(51) Int Cl.:
*G01N 21/17* *(2006.01)*    *A61B 5/00* *(2006.01)*

(21) Application number: **12006018.1**

(22) Date of filing: **27.07.2009**

(54) **Thermoacoustic imaging with quantitative extraction of an absorption image**

Thermoakustische Bildgebung mit quantitativer Extraktion einer Absorptionsabbildung

Imagerie themoacoustique avec extraction quantitative d'une image d'absorption

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.07.2008 PCT/EP2008/006142**
**29.06.2009 PCT/EP2009/004687**

(43) Date of publication of application:
**28.11.2012 Bulletin 2012/48**

(62) Document number(s) of the earlier application(s) in
accordance with Art. 76 EPC:
**09009683.5 / 2 148 183**

(73) Proprietor: **Helmholtz Zentrum München Deutsches**
**Forschungszentrum für Gesundheit**
**und Umwelt (GmbH)**
**85764 Neuherberg (DE)**

(72) Inventors:
- **Razansky, Daniel**
  **D-80686 München (DE)**
- **Ntziachristos, Vasilis**
  **D-82166 Gräfelfing (DE)**

(74) Representative: **Linsmeier, Josef**
**Wallinger Ricker Schlotter Tostmann**
**Patent- und Rechtsanwälte**
**Zweibrückenstrasse 5-7**
**80331 München (DE)**

(56) References cited:
**US-A1- 2002 193 678     US-B1- 6 567 688**

**EP 2 527 815 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

**Description**

Subject of the invention

The invention relates to imaging devices being configured for optoacoustic or thermoacoustic imaging of an object. Furthermore, the invention relates to imaging methods for optoacoustic or thermoacoustic imaging of an object. In particular, the invention relates to devices and methods for quantitative three-dimensional sensing and imaging of target tissue biomarkers, in particular in clinical, small animal and small organism imaging applications using multiple-wavelength illumination.

Technical background

Multi-spectral optoacoustic tomography (MSOT) is a method capable of resolving chromophoric agents with molecular specificity through several millimeters to centimeters of tissue. The technique is based on the optoacoustic phenomenon, i.e. the generation of acoustic waves due to thermoelastic expansion caused by absorption of ultra-short optical pulses. Optoacoustic interrogation of living tissues is recently drawing vast attention due to its ability of preserving scattering-free spatial resolution deep in highly scattering tissues while providing rich contrast characteristic of optical spectrum. Over the last decade optoacoustics has been considered for tissue imaging, mainly for resolving vascular contrast and the corresponding physiological changes, in particular oxy- and deoxy-hemoglobin, superficial vascular anatomy, brain lesion and functional cerebral hemodynamic changes, blood volume and oxygen consumption changes and the associated dynamic and functional neuronal activities.

The main difficulty arising from 3D optoacoustic imaging are long acquisition times associated with recording signals from multiple spatial projections.

In order to effectively propagate and reach the detector's surface, the generated optoacoustic waves need an acoustically-matched medium. As an example, an arrangement of the object to be imaged and a detector element in a container with water have been proposed by D. Razansky and V. Ntziachristos ("Med. Phys." Vol. 34, 2007, pp. 4293-4301).

If, for instance, a living animal being imaged resides in water, variety of hygienic, epidemiological and general handling issues readily arise. Moreover, many areas of the body become inaccessible for imaging unless the animal is submerged entirely into the liquid, which requires specialized breathing accessories and further complicates the overall imaging configuration and abilities for fast and reliable data acquisition.

The generated optoacoustic responses are generally weak and the SNR is low, problems that are usually solved by multiple signal averaging, which only further complicates matters and makes imaging challenging for most applications, especially those dealing with living subjects. For instance, one could obtain a rough estimate on the order of magnitude of a typical optoacoustic disturbance by considering a simplified one-dimensional case of a short pulsed beam impinging upon absorbing half space. Under heat and temporal stress confinement conditions, pressure-rise distribution $P(r)$ can be expressed as,

$$P(r) = \frac{\beta C_s^2}{C_P} \mu_a(r)\phi(r) = \Gamma \mu_a(r)\phi(r)$$

where the typical parameters for biological tissue are $\beta = 3 \cdot 10^{-4}$ ($°C^{-1}$) for the thermal expansion coefficient; $C_s = 15 \cdot 10^{-4}$ (cm s$^{-1}$) for the speed of sound; $C_p = 4.186$ (J g$^{-1}$ $°C^{-1}$) for the specific heat capacity at constant pressure; and $\mu_a = 0.3$ cm$^{-1}$ for the optical absorption coefficient (see K. V. Larin et al. in "Journal of Physics D-Applied Physics" vol. 38(15), 2005, p. 2645-2653). In a typical imaging scenario, illumination of tissue with maximal permissible fluence of $\phi = 20$ mJ/cm$^2$ will only result in optoacoustic disturbances with less than 1 kPa magnitude on the tissue surface light is incident upon, which will translate into an order of magnitude lower detectable pressure variations on the detector's surface. If deep tissue imaging is of interest, the pressure variations will be further affected by light attenuation and acoustic dispersions, which will bring the signals down by another order of magnitude and more so that only few Pascals are available for detection. Finally, when considering multispectral optoacoustic tomography (MSOT) data acquisition, in which tomographic data is recorded at several different wavelengths, 3D image acquisition times readily become unrealistic.

From the practical imaging perspective and SNR considerations it is desirable to increase detection sensitivity rather than reduce the thermal noise by using multiple measurements/averages.

An imaging device for optoacoustic or thermoacoustic imaging is disclosed in US 6, 567, 688 B.

Objective of the invention

The objective of the invention is to provide improved imaging devices for optoacoustic imaging of an object avoiding disadvantages and limitations of conventional techniques. In particular, the improved imaging device is to be capable of high performing and quantitative optoacoustic imaging. Furthermore, the objective of the invention is to provide improved imaging methods for optoacoustic imaging of an object. Of particular interest is the development of fast-acquisition imaging approaches for whole body imaging of model organisms and disease biomarkers in-vivo that will enable studying fast-varying biological phenomena, real-time visualization of biomarker distribution, pharmacokinetics, treatment responses etc..

## Summary of the invention

The above objectives are achieved with imaging devices and/or methods comprising the features of the independent claims, respectively. Preferred embodiments of the invention are defined in the dependent claims. Furthermore, the above objectives are achieved with imaging devices and/or methods described in the present description.

According to a first aspect of the invention, an imaging device being configured for optoacoustic imaging of an object comprises an illumination device including optical components arranged for a pulsed illumination of the object, a detector device arranged for a detection of acoustic signals created in the object, and a container device arranged for accommodating the detector device, the object and a matching transmission medium (in particular matching fluid). The container device accommodates a holding device which is capable of positioning the object in the container. The imaging device is configured such that the holding device with the object and the illumination and detector devices are movable relative to each other.

According to a second aspect of the invention, an imaging method of optoacoustic imaging an object comprises the steps of positioning the object with a holding device in a container device, which includes a matching transmission medium and a detector device, illuminating the object via optical components of an illumination device, detecting acoustic signals created in the object in response to the illumination with the detector device, and reconstructing a tomographic image of the object by analyzing the acoustic signals. During the detection of the acoustic signals, the holding device with the object and the illumination and detector devices are moved relative to each other.

According to the invention, at least some of the optical components of the illumination device are arranged in the container device. Preferably, the optical components of the illumination device are arranged such that the object can be illuminated from different directions. The optical components comprise any element which is adapted for deflecting and/or focusing a light beam directed to the object. As an advantage, a patterned illumination of a predetermined region of interest, in particular a predetermined detection plane in the object can be obtained by distributing the optical components on different sides of the object. The optical components can be positioned in immediate neighborhood of the holding device accommodating the object.

In a preferred embodiment the illumination pattern is a line illumination perpendicular to the object axis. The line illumination can be projected from multiple angles surrounding the object and their intensity can be varying across the line in order to create a homogenous or close to homogenous light delivery around the object's surface. Contrary to conventional techniques, distortions by a container wall or other parts of the experimental setup can be avoided and the illumination intensity can be essentially increased by exclusively concentrating the illumination power or energy on the region of interest.

As the acoustic signals are detected, the object is moved with the holding device relative to the optical components of the illumination device or, alternatively, the optical system scans the pattern on the object. With this movement (scanning movement), the region of interest, in particular the detection plane is shifted through the object. Generally, the movement can comprise a rotation and/or a translation.

Furthermore, according to the invention, the detector device comprises an array of detector elements which is arranged in the container device. While a single detector can be used, which is time-shared at multiple position, it is an advantage to employ a detector array so that optoacoustic responses are collected at different locations simultaneously. The array of detector elements comprises any device which includes multiple detector elements at predetermined fixed positions. In the preferred embodiment, the detector elements are distributed along a predetermined straight or curved line (or plane or curved surface) in space, in correspondence to the line (or surface) illumination pattern established, so that the detectors detect from the volume illuminated. The detector elements can be arranged side by side adjacent to each other and/or with mutual spacings. During the detection of the acoustic signals, the object may be moved with the holding device relative to the array of detector elements (scanning movement) or the detection, the illumination or both may be moved relative to the object.

The proposed solution utilizes parallel detection configuration using multiple large-area focused ultrasonic detection elements for fast data acquisition. Both illumination and detection topologies are optimized for fast data acquisition and real-time image reconstruction. Preferably, the optoacoustic data is sequentially collected at multiple excitation wavelengths in order to construct images of the target representing anatomical and functional information as well as distribution of intrinsic or extrinsically administered biomarkers and contrast agents. The detectors can be effectively focused along a line (cylindrically focused perpendicular to the objects axis in analogy to the illumination pattern). However other patterns can be foreseen implemented, for example spherically-focused elements or arrangements or unfocused elements for collection of three-dimensional data.

Preferably, the invention is used for imaging tissue of small animals, tissue of mesoscopic size i.e. tissue having a typical dimension in the range of 100 $\mu$m to 10 cm or more, e. g. 100 $\mu$m to 5 cm, in particular from 1 mm to 3 cm, or tissue or a tissue component of a human body (or an animal body having a size comparable with a human body).

Preferably, the images of the object reconstructed during the scanning movement comprise 2D images collected and reconstructed in real time. Imaging in real time means that a 2D single or multi wavelength image is collected within a time below 2 s, e. g. below 1 s, in particular below 500 ms. A 3D image can be constructed based on a series of 2D images of the object along the detection planes scanned during the scanning movement. The series of 2D images is preferably collected by stepwise adjusting the object relative to a detection plane of the detector device, illuminating the object with pulsed illumination light and detecting of the acoustic signals with the adjusted object position, wherein the illuminating and detecting steps are repeated at least twice, e. g 3, 4, 5, or more times with different wavelengths of the illumination light. For constructing the 3D image, a series of at least 5 slices, e. g. 20 slices, preferably 100 slices, like 200 slices or even more is collected.

According to a preferred embodiment of the invention, the detector device comprises an array of focussed detector elements which are focussed into the predetermined region of interest, in particular the predetermined detection plane in the object. Advantageously, this results in an increased signal-to-noise-ratio of the signal collection. Focussed detector elements have a sensitivity profile with a main sensitivity direction (focussing direction). Preferably, the detector elements comprise at least one of piezoelectric film detector elements, and film detector elements adapted for interferometric signal recording.

According to a further preferred embodiment of the invention, the detector elements are arranged along an arc at least partially surrounding the object. The detector device is configured such that the arc-shaped (e. g. semicircle-shaped) array of detector elements at least partially surrounds the path of movement of the object and the detector array relative to each other. The detector elements are spanned along a detection plane, through which the object is scanned for 3D imaging.

Preferably, the imaging device provides a semicylindrical (180 degrees) detection geometry. The arc of detector elements spans 180 °. An advantage of this geometry is that it allows the object to be "immersed" in the matching medium and allow a better viewing angle (projections) by a (cylindrical) detection geometry.

As a further advantage, there are not particular limitations with regard to the type of optical components used, which preferably may comprise optical mirrors or optical fibers located in the container. Optical fibers are particularly preferred as optical fibers are capable of guiding the illumination light pulses with minimum losses towards the object. According to a further preferred embodiment of the invention, the optical fibers comprise a fiber bundle having multiple arms, e. g. at least three arms, preferably at least 5 arms, e. g. 10 arms or more. Preferably, the optical fibers are arranged at equally spaced angles along an azimuthal line surrounding the object. According to a further preferred embodiment of the invention, the optical components, preferably the optical fibers, are arranged for illuminating the object with a ring type pattern.

According to a further embodiment of the invention, the holding device comprises a rod or plate shaped holding element arranged for carrying the object. The object can be directly connected with the rod or plate shaped holding element. In this case, the object is directly immersed in the matching transmission medium in the container.

According to an alterative preferred embodiment of the invention, the holding device comprises a membrane arranged for accommodating the object. The membrane separates the object from the matching transmission medium. The object has no direct contact with the matching transmission medium. Preferably, the membrane is acoustically and optically matched for illuminating the object and detecting the acoustic signals. Acoustic matching means that acoustic reflections at the membrane's surface are minimized. Optical matching means that the membrane is made of a material being optically transparent for the wavelengths of the illumination / energy deposition. Advantageously, the membrane is made of at least one of polyvinyl chloride plastisol (PVCP), polyethylene and latex. For minimizing acoustic and optical losses, the membrane has a thickness below 5 mm, preferably below 1 mm e. g. below 200 $\mu$m.

This inventive design incorporating the acoustically and optically matched membrane allows a separation of the imaged object from the matching transmission medium into which the detector device is embedded for optoacoustic detection. This allows practical imaging applications without direct contact with the matching transmission medium, e. g. water.

Preferably, the holding device comprises a frame shaped holding element arranged for carrying the membrane. The membrane can be fixed to the frame shaped holding element such that a bag (or pocket, non-rigid container) is formed accommodating the object to be imaged. The membrane with the accommodated object can be immersed into the matching transmission medium in the container such that an upper side of the object is exposed to the surrounding gas atmosphere, e. g. air. The frame shaped holding element can be shaped in different shapes, for example a half circular tube so that the center of mass of the object or the central axis of the object lie in the center of a curved ultrasonic detection array, also shaped in a partial circular geometry. This arrangement can lower the object within the matching transmission medium, versus placing it on top of the matching transmission medium, thus allowing a better coverage of the acoustic responses generated from within the object by the corresponding surrounding multi-element detector. As a further advantage, the frame shaped holding element can be movable. For conducting the scanning movement, the frame shaped holding element can be moved along a guide rail in the container.

As a further advantage, utilizing the membrane allows imaging of living objects, like e. g. animals. Accordingly, the imaging device can be provided with an anaesthesia device arranged for subjecting the object to an anaesthesia treatment.

The imaging device of the invention is adapted for conducting the imaging and image reconstructing methods described in WO 2010/009747 and WO 2011/000389. To this end, the illumination device is adapted for illuminating the object with different wavelengths. Preferably, the illumination device comprises laser source with wavelength tuning. Particularly preferred is a tunable optical parametric oscillator (OPO) laser including a tiltable non-linear crystal for wavelength scanning. It has been found, that this OPO device allows rapid wavelength changes adapted for the inventive real time imaging.

In summary, this application is filed in continuation of WO 2010/009747 and aims at providing an efficient method and system in particular for non-contact multispectral optoacoustic tomography of whole body or localized regions of small animals in real time. To this end, the imaging device (signal collecting device) comprises the illumination device including an electromagnetic energy source, e. g. pulse laser source, and an acoustic signal detector device with a detector array and/or a single detector, including at least one acoustic detection element. Other sources include sources with frequencies in the kHz to GHz range, i.e. radiofrequencies and frequencies around them (see below). Preferably, the acoustically-matched membrane is provided separating the imaged object from a liquid medium where the at least one acoustic detection element is located. Advantageously, the membrane allows for the object to be imaged without being immersed into the liquid medium. According to the examples described below, the object is placed on top of a transparent membrane and illuminated with a laser source. The membrane is preferably made of optically transparent material that also does not significantly alter propagation of sound, e.g. acoustically-matched membrane. In this way, an efficient transmission of both laser radiation and the induced optoacoustic response is facilitated. The membrane also ensures that the imaged object can be conveniently placed for imaging without direct contact with water.

According to further independent solutions, the above objectives are achieved with imaging devices and/or methods modified according to the following aspects of the invention. With the following modifications, some inventive features of the embodiments described above are replaced by modified features which in combination again may solve the above objectives of the invention.

According to a further aspect of the invention, the multi-element array can be replaced by a single focused or unfocused detector element that will be scanned in the vicinity or around the object in order to acquire spatially resolved or tomographic data. This typically would represent a non-real time embodiment, and it might be useful in order to image a localized area of an animal, for instance containing a tumor. In that case, by linear scanning of a spherically focused detector element along one or two dimensions using translation stage/s, 2D or 3D images of the region of interest can be obtained by using e.g. delay-and-sum algorithm known from ultrasonic imaging. The illumination of the object is adjusted accordingly so that the light energy concentrates only in the region of interest. Using the single detector element is preferably combined with accommodating the object in the membrane.

According to a yet further aspect of the invention, the movement of the holding device relative to the optical components and the detector device can be replaced by a movement of the optical components and the detector device relative to the holding device, or combined movements of the optical components and the detector device and the holding device relative to each other.

According to a yet further aspect of the invention, the imaging with an optical generation of acoustic waves can be replaced by thermoacoustic imaging. In this case, the laser source of the illumination device is replaced by a generator device including an electromagnetic pulse source with frequencies e. g. in the radiofrequency range. Furthermore the optical components would be replaced by irradiation components, like antennas or wave guides, which can be arranged outside the tank or in a sealed manner in the tank of the container device.

Brief description of the drawings

Further details and advantages of the invention are described in the following with reference to the attached drawings, which show in:

| | |
|---|---|
| Figures 1 and 2: | schematic illustrations of a preferred embodiment of the inventive imaging device; |
| Figure 3: | a schematic illustration of further details of the first embodiment of the inventive imaging device; |
| Figure 4: | details of an array of detector elements used in the inventive imaging device; |
| Figure 5: | a graphical representation of a wavelength dependence of molar extinction of AF750 and common tissue chromophores; and |
| Figure 6: | a schematic illustration of a further preferred embodiment of the inventive imaging device. |

Preferred embodiments of the invention

Preferred embodiments of the invention are described in the following with exemplary reference to the structure and operation of the imaging device. Further details of the imaging and image reconstructing methods are implemented as described in the applications WO 2010/009747 and WO 2011/000389. These applications are introduced into the present specification by reference, in particular with regard to the quantitative three-dimensional sensing and imaging of target tissue biomarkers and analyzing the acoustic signals, in particular in clinical, small animal and small organism imaging applications using multiple-wavelength illumination. The introduction of endogenous or exogenous reporter agents with molecular specificity, such as fluorescent proteins and probes, further allows the propagation of this technique towards molecular imaging applications. In this case a promising approach is the use of multi-spectral illumination in order to differentiate specific spectral signatures of key reporter agents over the background tissue absorption. Combined, imaging of physiological and molecular markers using optoacoustics has the potential to high resolution photonic imaging, through depths that go significantly beyond the capabilities of modern microscopy.

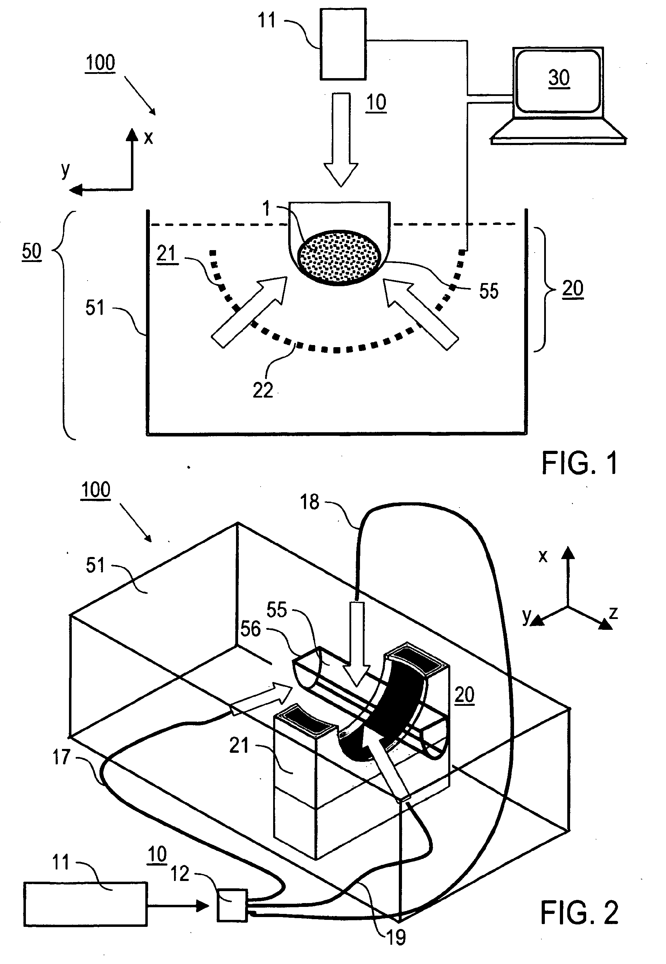

Figure 1 illustrates a preferred embodiment of the inventive imaging device 100 including an illumination device 10, a detector device 20, a control device 30, and a container device 50 comprising a tank 51. The control device 30 is arranged for controlling the illumination device 10 and the detector device 20 and for conducting the steps of the image reconstructing methods. The tank 51 contains a matching transmission medium 53, e.g. water or oil. The illumination device 10 comprises a laser source 11 and optical components 17, 18, like e. g. optical fibers or mirrors arranged in the water tank 51. The container device 50 further includes a holding device comprising a transparent membrane 55. The object 1, like e. g. a mouse has a main extension (longitudinal direction, object axis) representing e. g. the z-direction.

According to Figure 1, the object 1 illuminated with the laser source 11 is placed on top of the transparent membrane 55. The membrane 55 is made of optically transparent material that also does not significantly alter propagation of sound, e.g. acoustically-matched membrane made of polyvinyl chloride plastisol (PVCP) or thin films of polyethylene. In this way, an efficient transmission of both laser radiation and the induced optoacoustic response is facilitated. The membrane 55 also ensures that the imaged object 1 can be conveniently placed for imaging without direct contact with water. The membrane 55 is placed in contact with the water tank 51, into which the detector array 21 is immersed.

The detector array 21 is e. g. a curved array with 64 acoustic detection elements 22, which form for example 180° arc that surrounds the object 1. In the example of Figure 1, all the acoustic detection elements 22 are cylindrically focused in the detection plane to allow for simultaneous collection of signals from a two-dimensional detection plane (drawing plane, x-y-plane). The imaging device 100 will therefore collect two-dimensional data in real time.

A schematic perspective view of the imaging device 100 according to a modified embodiment is presented in Figure 2. The illumination device 10 comprises a laser 11 and optical components 17, 18 and 19 fixedly arranged in the container 51 for illuminating the object 1 (not shown) in the bag-shaped membrane 55. The optical components 17, 18 and 19 comprise optical fibers into which light pulses emitted by the laser 11 are coupled using an optical coupler 12. The detector device 20 comprises an array 21 of detector elements integrated in a common block. The detector device 20 is fixedly positioned on the bottom of the container 51. Both the illumination device 10 and the detector device 20 are connected with a control device (not shown).

As an alternative, plane or curved mirrors could be used as optical components 17, 18 and 19 for illuminating the object from different sides. Light from a laser source could be coupled to the mirrors through an upper surface of the matching transmission medium 53 in the tank 51, so that distortions by the tank walls are suppressed.

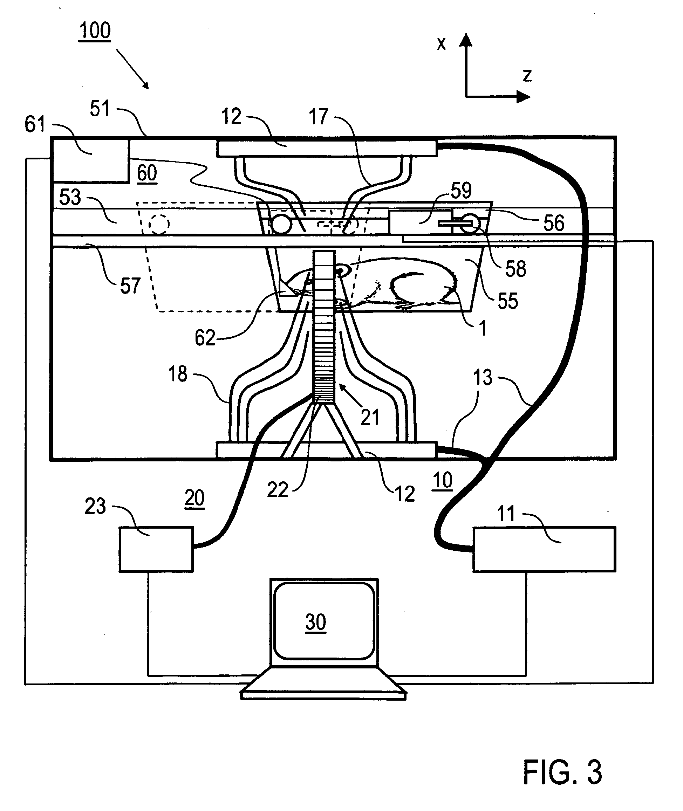

A holding device 55, 56 is arranged for positioning and moving the object relative to the optical components 17, 18 and 19 of the illumination device 10 and relative to the detector device 20. The movement is directed perpendicular to the detection plane, i. e. in z-direction. The holding device comprises the membrane 55 and a frame shaped holding element 56 to which the membrane 55 is attached. The frame shaped holding element 56 is supported by guide rail in the container device as further illustrated in Figure 3. The membrane 55 can provide a bag supporting the object from below as shown in Figure 1 or an envelope covering the object from multiple sides. In particular, the membrane may have a tube or hose shape. In this case, the complete membrane can be immersed into the matching transmission medium, while the ends thereof are closed or located above a surface of the matching transmission medium.

A schematic side view of the imaging device 100 according to a further modified second embodiment is presented in Figure 3. The illumination device 10 comprises a laser 11 being connected via light-guiding fibres 13 to optical couplers 12. The optical couplers 12 are connected with a plurality of fixedly arranged optical fibers 17, 18 the output ends of which facing to the object 1. The optical fibers 17, 18 are schematically illustrated with mutual distances. In practice, the optical fibers 17, 18 are arranged such the output ends thereof are located on a circle line surrounding the bag-shaped membrane 55 and the object 1.

To attain optimal quality of tomographic reconstructions, the light is shined upon the imaged object from multiple directions to ensure uniform illumination of the object at the imaging plane. This can be done by e.g. guiding the light by means of splitting a free-space beam or - as illustrated - using a fiber bundle. In the preferred embodiment, the bundle splits into 10 arms that are placed in the vicinity of the imaged object and illuminate it from 10 equally-spaced angles adding up to 360°. Optimally, the illuminating light forms a ring-type pattern on the object's surface. The effective width of this illumination ring is optimally adjusted to achieve maximal possible confinement of excitation light intensity within the imaged plane (cross-section) of the object. This can be done by for instance minimizing the light width to a minimal permissible value so that the safety limits of tissue exposure to pulsed laser radiation are met, e.g. the fluence on the tissue surface does not exceed 20 mJ/cm$^2$ at a wavelength of 750 nm as proposed in "American National Standards for the Safe Use of Lasers", ANSI Z136.1, American Laser Institute, 2000.

The detector device 20 comprises an array 21 of detector elements 22 being connected with a signal recorder 23 outside the container 51. The illumination device 10 and the detector device 20 are connected with the control device 30. The detector elements 22 are selected in view of the requirements for high resolution and quality optoacoustic tomographic reconstructions that directly correspond to the detection bandwidth, which should ideally be as large as possible. For this one could use ultrawideband ultrasound detection technologies, detectors including piezoelectric polymers, such as pVDF film detectors and Fabry-Perot interferometric detector elements that have already proven to be a potential tool for high-resolution optoacoustic imaging in vivo. Compared to the latter broadband approaches, PZT and other piezocomposite technologies can normally provide higher SNR and robustness, in exchange however for narrower bandwidth.

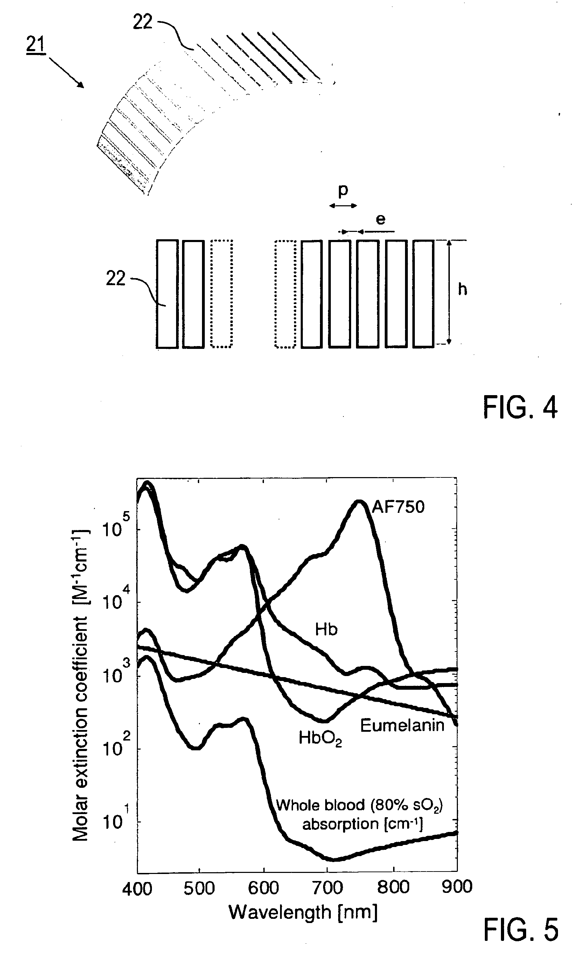

Preferably, the detector array 21 is a curved concave array with acoustic detector elements 22 as shown in Figure 4. As an example, the number of acoustic detector elements 22 is selected in the range of 64 to 256. The detector elements 22 form for example an 180° arc that surrounds the object 1. In example of Figure 4, each one of the strip-shaped acoustic detection elements (Pitch p=0.98mm; Height h=15mm; Inter element spacing e=0.1mm) is cylindrically or spherically focused in the detection plane to allow for simultaneous collection of signals from a two-dimensional detection (imaging) plane in real time. For example, given pulsed laser source with 10 Hz repetition frequency, in plane 2D data can be collected every 100 ms per single wavelength. The magnitude of the recorded optoacoustic signals is normally directly proportional to the detector's area while the noise floor decreases only as a function of square root of the number of averages or measurements. As a result, a single focused element 22 will for instance be way more superior in terms of SNR to multi-element array spread over the same detection area. The acoustic focusing if thus used here in order to increase detection area and sensitivity, in exchange for compromising image quality and resolution along dimensions where focusing is performed. This solution comprises therefore a sort of compromise between acquisition speed and imaging performance.

A guide rail 57 is located in the container 51 for carrying the frame shaped holding element 56 as shown in Figure 3. The frame shaped holding element 56 is provided with wheels 58 running on the guide rail 57 and an electric motor 58 connected with the control device 30. The membrane 55 with the object 1 is moved in z-direction by actuating the electric motor 58. As an example, the object can be scanned from the illustrated position to the position shown with dashed lines. For implementing a movement of the optical components and the detector device relative to the holding device, a guide rail and a drive unit can be arranged in the container, e. g. on a bottom surface thereof.

Figure 3 further illustrates an anesthesia device 60, which comprises an anesthesia gas source 61 and a mask 62 to be set on the head of a living object under investigation, like a mouse. The anesthesia device 60 is structured as it is known from animal holders used e. g. for X-Ray CT or MRI investigations.

For conducting the inventive imaging method, the object 1 is positioned on the membrane 55 in the container 51. As an example, a nude (hairless) mouse subjected to an anesthesia is set on the membrane 55. The membrane is placed in contact with the matching transmission medium 53 into which the ultrasonic detection array 21 is immersed.

Subsequently, a three-dimensional data acquisition is achieved via scanning the imaged object 1 across the illumination-detection plane that remains stationary or vice versa. Image reconstruction can be achieved e.g. by using cylindrical or spherical Radon-based back-projection algorithm. In the preferred embodiment of multispectral imaging applications, the laser wavelength is scanned at each imaging plane so that the full set of tomographic data is collected at all the required wavelengths prior to moving the object or imaging plane to another location. This is done since some biomarkers may present only small variations of the optical absorption over highly absorbing background, in which case even small quantification inaccuracies may lead to uninterpretable results.

In the preferred embodiment, fast wavelength scanning is performed by means of the tunable optical parametric oscillator (OPO) laser. For instance, in order to attain highly sensitive detection of AlexaFluor750™ (AF750) molecular probe in tissue, whose characteristic spectrum is shown in Figure 5, one could perform optoacoustic imaging with several wavelengths around the peak extinction of the probe, e. g. with 5 wavelengths (710, 730, 750, 770, 790 nm). With pulse repetition rate of 10Hz, i.e. each pulse is fired every 100ms, the output wavelength of the OPO laser will be repetitively scanned in the range 710-790 nm (with the speed of 20nm per 100ms) by continuously shifting the angle of the BBO ($BaB_2O_4$) nonlinear crystal with the respect to the pumping beam direction inside the OPO. In this case, a full set of multispectral tomographic data is acquired every 500 msec. The approach therefore ensures robust and reproducible results since the full set of multispectral data is acquired while the object remains fully stationary with respect to both illuminating light source/s and detector/s.

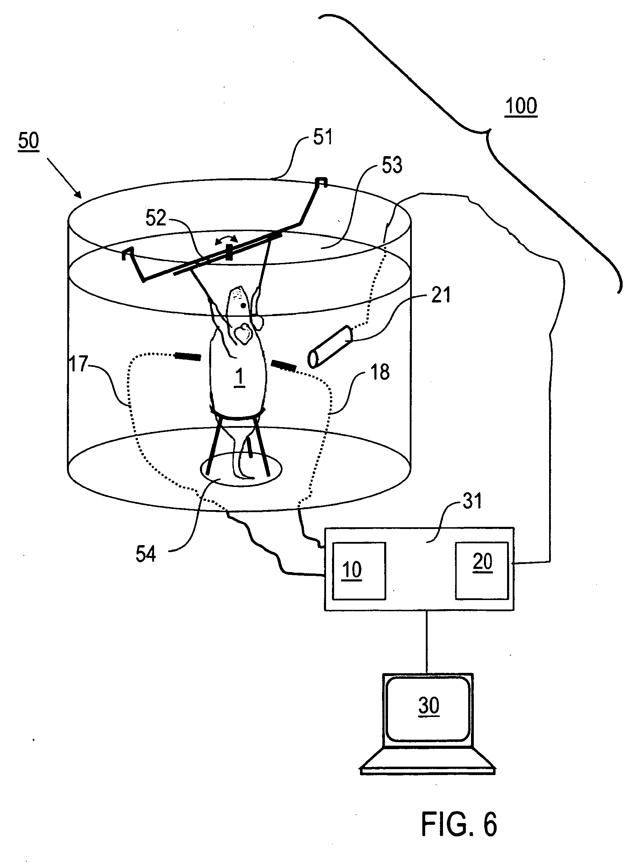

Figure 6 illustrates another preferred embodiment of the inventive imaging device 100 for biomedical imaging of mesoscopic-size objects and small animals, like mice or other rodents, flies, fishes, worms, non-human animal embryos. The imaging device 100 comprises the illumination device 10, the detector device 20 and the control device 30. Furthermore, the container device 50 is provided, which comprises the tank 51 and holding elements 52, 54 which are adapted for positioning components of the imaging device 100. The tank 51 contains the matching transmission medium 53, e.g. water or oil. The object to be investigated (living mouse 1) is positioned on the lower part 54 of the rod or plate shaped holding element.

Preferably, the illumination light comprises at least one characteristic wavelength of at least 100 nm, preferably at least 400 nm, particularly preferred at least 650 nm, and below 5000 nm, preferably below 1200 nm, particularly preferred below 850 nm. For this embodiment, the illumination device 10 includes a laser source device being adapted for emitting light pulses, preferably in the visible or near-infrared spectra. The light pulses preferably have a duration below 1μs, particularly preferred below 50 ns.

The illumination device 10 and the detector device 20 are partially integrated in a casing 31, that is arranged outside the container device 50. The illumination device 10 comprises a pulsed laser source whose light is directed to the mouse 1 from two opposite directions 17,18 by e.g. using optical mirrors or fibers. The detector device 20 comprises an array 21 of acoustic detector elements. The detector device 20 is arranged in the neighbourhood of the holding element 52 with the mouse 1. Advantageously, there are no particular restrictions with regard to the position of the detector device 20. The preferred location however will be as close as possible to the object to obtain measurements with high signal-to-noise ratios. For implementing the above image reconstruction, it is only necessary to have information on the location of the array of detector elements relative to the object (mouse 1).

The embodiments schematically illustrated in the figures are not restricted to the investigation of small animals. Alternatively, other biological objects can be imaged, e.g. human beings or larger animals or parts thereof. As an example, tank 51 can be adapted for accommodating a part of a human patient instead of the mouse 1.

While the invention is described above with reference to the use of an array of detector elements, for implementing embodiments using a single detector element, the array is to be replaced by a combination of the single detector element and at least one translation stage.

The features of the invention in the above description, the drawings and the claims can be of significance both individually as well in combination for the realisation of the invention in its various embodiments.

## Claims

1. Imaging device (100) being configured for optoacoustic imaging of an object (1), comprising:

   - an illumination device (10) including optical components arranged for illuminating the object (1) with light,
   - a detector device (20) arranged for detecting acoustic signals created in the object (1),
   - a container device (50) including a tank (51) arranged for accommodating the detector device (20), the object (1) and a matching transmission medium (53), and
   - a holding device (52, 54, 55) for positioning the object (1) in the container device (50), wherein
   - the detector device (20) comprises an array of detector elements and
   - the holding device (52, 54, 55) with the object (1) and/or the illumination device (10) and/or the detector device (20) are movable relative to each other, wherein the movement comprises a translation,
   **characterized in that**
   - the illumination device (10) is movable relative to the object (1) or the detector device (20) and the illumination device (10) are movable relative to the object (1) and/or
   - the holding device comprises a membrane (55) arranged for accommodating the object (1), wherein the membrane separates the object (1) and the matching transmission medium (53) from each other.

2. Imaging device according to claim 1, wherein

   - the object (1) can be moved with the holding device relative to the array of detector elements.

3. Imaging device according to claim 1 or 2, wherein

   - the detector device (20) comprises an array of focused detector elements which are focused into a common detection plane in the object.

4. Imaging device according to one of the foregoing claims, wherein

- the detector elements are arranged along an arc at least partially surrounding the object (1).

5. Imaging device according to one of the foregoing claims, wherein

- the detector elements are arranged along a semicircle-shaped or semi-cylindrical arc.

6. Imaging device according to one of the foregoing claims, wherein

- the detector elements are distributed along a curved line and/or along curved surface.

7. Imaging device according to one of the foregoing claims, wherein

- the optical components comprise optical mirrors or optical fibers located in the tank (51), and/or
- the optical components are arranged for illuminating the object (1) with a ring type pattern.

8. Imaging device according to claim 7, wherein

- the optical fibers comprise a fiber bundle having multiple arms which are arranged at equally spaced angles adding up to 360 °.

9. Imaging device according to one of the foregoing claims, wherein the holding device comprises

- a rod or plate shaped holding element (52, 54) arranged for carrying the object (1).

10. Imaging device according to one of the foregoing claims, wherein the membrane has at least one of the features

- the membrane (55) is acoustically and optically matched for illuminating the object (1) and detecting the acoustic signals,
- the membrane (55) is made of at least one of polyvinyl chloride plastisol, polyethylene and latex, and
- the membrane (55) has a thickness below 1 mm.

11. Imaging device according to one of the foregoing claims, wherein the holding device comprises

- a frame shaped holding element (56) arranged for carrying the membrane (55).

12. Imaging device according to claim 11, wherein

- the frame shaped holding element (56) is movable along a guide rail (57) in the tank (51).

13. Imaging method of optoacoustic imaging of an object (1), comprising the steps of

- positioning the object (1) with a holding device (52, 54, 55) in a tank (51) of a container device (50), in which tank (51) a matching transmission medium (53) and a detector device (20) are included,
- illuminating the object (1) via optical components of an illumination device (10),
- detecting of acoustic signals created in the object (1) with an array of detector elements of the detector device (20), wherein the object (1) with the holding device (52, 54, 55) and/or the illumination device (10) and/or the detector device (20) are moved relative to each other during the detecting of the acoustic signals, wherein the movement comprises a translation, and
- reconstructing an image of the object (1) by analyzing the acoustic signals,
**characterized in that**
- the illumination device (10) is moved relative to the object (1) or the detector device (20) and the illumination device (10) are moved relative to the object (1) and/or
- the holding device comprises a membrane (55) in which the object (1) is accommodated, wherein the membrane separates the object (1) and the matching transmission medium (53) from each other.

14. Imaging method according to 13, comprising

- stepwise adjusting the object relative to a detection plane of the detector device, illuminating the object with pulsed illumination light or irradiating the object with electromagnetic energy and detecting of the acoustic signals with the adjusted object position, wherein the illuminating or irradiating and detecting steps are repeated at least twice with different wavelengths of the illumination light or electromagnetic energy, respectively.

15. Imaging device (100) being configured for thermoacoustic imaging of an object (1), comprising:

- a generator device including irradiation components arranged for irradiating the object (1) with electromagnetic energy, in particular in the radiofrequency range,
- a detector device (20) arranged for detecting acoustic signals created in the object (1),
- a container device (50) including a tank (51) arranged for accommodating the detector device (20), the object (1) and a matching transmission medium (53), and
- a holding device (52, 54, 55) for positioning the object (1) in the container device (50), wherein
- the detector device (20) comprises an array of detector elements and
- the holding device (52, 54, 55) with the object (1) and/or the generator device and/or the detector device (20) are movable relative to each other, wherein the movement comprises a translation,
**characterized in that**
- the generator device (10) is movable relative to the object (1) or the detector device (20) and the generator device (10) are movable relative to the object (1) and/or
- the holding device comprises a membrane (55) arranged for accommodating the object (1), wherein the membrane separates the object (1) and the matching transmission medium (53) from each other.

16. Imaging method of thermoacoustic imaging of an object (1), comprising the steps of

- positioning the object (1) with a holding device (52, 54, 55) in a tank (51) of a container device (50), in which tank (51) a matching transmission medium (53) and a detector device (20) are included,
- irradiating the object (1) with electromagnetic energy, in particular in the radiofrequency range, via irradiation components of a generator device,
- detecting of acoustic signals created in the object (1) with an array of detector elements of the detector device (20), wherein the object (1) with the holding device (52, 54, 55) and/or the generator device and/or the detector device (20) are moved relative to each other during the detecting of the acoustic signals, wherein the movement comprises a translation, and
- reconstructing an image of the object (1) by analyzing the acoustic signals,
**characterized in that**
- the generator device (10) is moved relative to the object (1) or the detector device (20) and the generator device (10) are moved relative to the object (1) and/or
- the holding device comprises a membrane (55) in which the object (1) is accommodated, wherein the membrane separates the object (1) and the matching transmission medium (53) from each other.

**Patentansprüche**

1. Bildgebungsvorrichtung (100), welche für die optoakustische Bildgebung eines Objekts (1) ausgestaltet ist, enthaltend:

- eine Beleuchtungsvorrichtung (10), welche optische Komponenten, welche zur Beleuchtung des Objekts (1) mit Licht ausgestaltet sind, enthält,
- eine Detektorvorrichtung (20), die zum Detektieren von im Objekt (1) erzeugten akustischen Signalen ausgestaltet ist, und
- eine Behältervorrichtung (50), welche einen Behälter (51), der zum Aufnehmen der Detektorvorrichtung (20), des Objekts (1) und eines Anpassungstransmissionsmediums (53) ausgestaltet ist, enthält, und
- eine Haltevorrichtung (52, 54, 55) zum Positionieren des Objekts (1) in der Behältervorrichtung (50), wobei
- die Detektorvorrichtung (20) eine Anordnung an Detektorelementen aufweist und
- die Haltevorrichtung (52, 54, 55) mit dem Objekt (1) und/oder die Beleuchtungsvorrichtung (10) und/oder die Detektorvorrichtung (20) relativ zueinander bewegbar sind, wobei die Bewegung eine Translation beinhaltet, **dadurch gekennzeichnet, dass**
- die Beleuchtungsvorrichtung (10) relativ zum Objekt (1) bewegbar ist oder die Detektorvorrichtung (20) und die Beleuchtungsvorrichtung (10) relativ zum Objekt (1) bewegbar sind und/oder
- die Haltevorrichtung eine Membran (55) aufweist, welche zum Aufnehmen des Objekts (1) ausgestaltet ist, wobei die Membran das Objekt (1) und das Anpassungstransmissionsmedium (53) voneinander trennt.

2. Bildgebungsvorrichtung nach Anspruch 1, wobei

- das Objekt (1) mit der Haltevorrichtung relativ zur Anordnung an Detektorelementen bewegt werden kann.

3. Bildgebungsvorrichtung nach Anspruch 1 oder 2, wobei

- die Detektorvorrichtung (20) eine Anordnung fokussierter Detektorelemente aufweist, welche in einer gemeinsamen Detektionsebene im Objekt fokussiert sind.

4. Bildgebungsvorrichtung nach einem der vorhergehenden Ansprüche, wobei

- die Detektorelemente entlang eines Bogens, welcher zumindest teilweise das Objekt (1) umgibt, angeordnet sind.

5. Bildgebungsvorrichtung nach einem der vorhergehenden Ansprüche, wobei

    - die Detektorelemente entlang eines halbkreisförmigen oder halbzylindrischen Bogens angeordnet sind.

6. Bildgebungsvorrichtung nach einem der vorhergehenden Ansprüche, wobei

    - die Detektorelemente entlang einer Bogenlinie oder einer gekrümmten Fläche verteilt sind.

7. Bildgebungsvorrichtung nach einem der vorhergehenden Ansprüche, wobei

    - die optischen Komponenten optische Spiegel oder optische Fasern, welche sich im Behälter (51) befinden, enthalten, und/oder
    - die optischen Komponenten zum Beleuchten des Objekts (1) mit einem ringartigen Muster ausgebildet sind.

8. Bildgebungsvorrichtung nach Anspruch 7, wobei

    - die optischen Fasern ein Faserbündel enthalten, das mehrere Arme besitzt, welche in Winkeln von gleichem Abstand, die sich zu 360° summieren, angeordnet sind.

9. Bildgebungsvorrichtung nach einem der vorhergehenden Ansprüche, wobei die Haltevorrichtung enthält:

    - ein stab- oder plattenförmiges Halteelement (52, 54), welches zum Tragen des Objekts (1) ausgestaltet ist.

10. Bildgebungsvorrichtung nach einem der vorhergehenden Ansprüche, wobei die Membran mindestens eines der Merkmale besitzt

    - die Membran (55) ist akustisch und optisch für das Beleuchten des Objekts (1) und das Detektieren der akustischen Signale angepasst,
    - die Membran (55) ist aus mindestens einem der Stoffe Polyvinylchlorid-Plastisol, Polyethylen und Latex hergestellt, und
    - die Membran (55) besitzt eine Dicke unterhalb von 1 mm.

11. Bildgebungsvorrichtung nach einem der vorhergehenden Ansprüche, wobei die Haltevorrichtung enthält

    - ein rahmenförmiges Halteelement (56), welches zum Tragen der Membran (55) ausgestaltet ist.

12. Bildgebungsvorrichtung nach Anspruch 11, wobei

    - das rahmenförmige Halteelement (56) entlang einer Führungsschiene (57) im Behälter (51) bewegbar ist.

13. Bildgebendes Verfahren zur optoakustischen Bildgebung eines Objekts (1), welches die Schritte enthält

    - Positionieren des Objekts (1) mit einer Haltevorrichtung (52, 54, 55) in einem Behälter (51) einer Behältervorrichtung (50), wobei im Behälter (51) ein Anpassungstransmissionsmedium (53) und eine Detektorvorrichtung (20) beinhaltet werden,
    - Beleuchten des Objekts (1) über optische Komponenten einer Beleuchtungsvorrichtung (10),
    - Detektieren eines akustischen Signals, welches im Objekt (1) erzeugt wird, mit einer Anordnung an Detektorelementen der Detektorvorrichtung (20), wobei das Objekt (1) mit der Haltevorrichtung (52, 54, 55) und/oder die Beleuchtungsvorrichtung (10) und/oder die Detektorvorrichtung (20) während des Detektierens akustischer Signale relativ zueinander bewegt werden, wobei die Bewegung eine Translation beinhaltet, und
    - Rekonstruieren eines Bildes des Objekts (1) durch Analysieren der akustischen Signale, **dadurch gekennzeichnet, dass**
    - die Beleuchtungsvorrichtung (10) relativ zum Objekt (1) bewegt wird oder die Detektorvorrichtung (20) und die Beleuchtungsvorrichtung (10) relativ zum Objekt (1) bewegt werden und/oder
    - die Haltevorrichtung eine Membran (55) aufweist, wobei das Objekt (1) aufgenommen wird, wobei die Membran das Objekt (1) und das Anpassungstransmissionsmedium (53) voneinander trennt.

14. Bildgebendes Verfahren nach Anspruch 13, enthaltend

    - ein stufenweises Einstellen des Objekts relativ zu einer Detektionsebene der Detektorvorrichtung, Beleuchten des Objekts mit gepulstem Beleuchtungslicht oder Bestrahlen des Objekts mit elektromagnetischer Energie und Detektieren der akustischen Signale in der eingestellten Objektposition, wobei die Stufen des Beleuchtens oder Bestrahlens und Detektierens mindestens zweimal mit unterschiedlichen Wellenlängen des Beleuchtungslichts beziehungsweise der elektromagnetischen Strahlung wiederholt werden.

15. Bildgebungsvorrichtung (100), welche für die thermoakustische Bildgebung eines Objekts (1) ausgestaltete ist, enthaltend:

- eine Generatorvorrichtung, welche Strahlungskomponenten beinhaltet, die zur Bestrahlung des Objekts (1) mit elektromagnetische Energie, insbesondere im Radiofrequenzbereich, ausgestaltet sind,
- eine Detektorvorrichtung (20), welche zum Detektieren von im Objekt (1) erzeugter, akustischer Signale ausgestaltet ist,
- eine Behältervorrichtung (50), welche einen Behälter (51), der zum Aufnehmen der Detektorvorrichtung (20), des Objekts (1) und eines Anpassungstransmissionsmediums (53) ausgestaltet ist, enthält, und
- eine Haltevorrichtung (52, 54, 55) zum Positionieren des Objekts (1) in der Behältervorrichtung (50), wobei
- die Detektorvorrichtung (20) eine Anordnung an Detektorelementen aufweist und
- die Haltevorrichtung (52, 54, 55) mit dem Objekt (1) und/oder die Generatorvorrichtung und/oder die Detektorvorrichtung (20) relativ zueinander bewegbar sind, wobei die Bewegung eine Translation beinhaltet,
**dadurch gekennzeichnet, dass**
- die Generatorvorrichtung (10) relativ zum Objekt (1) bewegbar ist oder die Detektorvorrichtung (20) und die Generatorvorrichtung (10) relativ zum Objekt (1) bewegbar sind und/oder
- die Haltevorrichtung eine Membran (55) aufweist, welche zum Aufnehmen des Objekts (1) ausgestaltet ist, wobei die Membran das Objekt (1) und das Anpassungstransmissionsmedium (53) voneinander trennt.

16. Bildgebendes Verfahren zur thermoakustischen Bildgebung eines Objekts (1), welches die Schritte enthält

- Positionieren des Objekts (1) mit einer Haltevorrichtung (52, 54, 55) in einem Behälter (51) einer Behältervorrichtung (50), wobei im Behälter (51) ein Anpassungstransmissionsmedium (53) und eine Detektorvorrichtung (20) beinhaltet werden,
- Bestrahlen des Objekts (1) mit elektromagnetischer Energie, insbesondere im Radiofrequenzbereich, über Bestrahlungskomponenten einer Generatorvorrichtung
- Detektieren von akustischen Signalen, welche im Objekt (1) erzeugt werden, mit einer Anordnung an Detektorelementen der Detektorvorrichtung (20), wobei das Objekt (1) mit der Haltevorrichtung (52, 54, 55) und/oder die Generatorvorrichtung und/oder die Detektorvorrichtung (20) während des Detektierens akustischer Signale relativ zueinander bewegt werden, wobei die Bewegung eine Translation aufweist, und
- Rekonstruieren eines Bildes des Objekts (1) durch Analysieren der akustischen Signale,
**dadurch gekennzeichnet, dass**
- die Generatorvorrichtung (10) relativ zum Objekt (1) bewegt wird oder die Detektorvorrichtung (20) und die Generatorvorrichtung (10) relativ zum Objekt (1) bewegt werden und/oder
- die Haltevorrichtung eine Membran (55) aufweist, wobei das Objekt (1) aufgenommen wird, wobei die Membran das Objekt (1) und das Anpassungstransmissionsmedium (53) voneinander trennt.

## Revendications

1. Dispositif d'imagerie (100) configuré de façon à imager un objet (1) de manière opto-acoustique, ledit dispositif d'imagerie comprenant :

- un dispositif d'éclairage (10) comprenant des composants optiques agencés pour éclairer l'objet (1) avec une lumière,
- un dispositif de détection (20) agencé pour détecter les signaux acoustiques créés dans l'objet (1),
- un dispositif de réception (50) comprenant une cuve (51) agencée pour recevoir le dispositif de détection (20), l'objet (1) et un milieu de transmission adéquat (53), et
- un dispositif de maintien (52, 54, 55) pour positionner l'objet (1) dans le dispositif de réception (50), dans lequel
- le dispositif de détection (20) comprend un ensemble d'éléments de détection, et
- le dispositif de maintien (52, 54, 55) avec l'objet (1) et/ou le dispositif d'éclairage (10) et/ou le dispositif de détection (20) peuvent être déplacés les uns par rapport aux autres, dans lequel le mouvement comprend une translation,
**caractérisé en ce que**
- le dispositif d'éclairage (10) peut être déplacé par rapport à l'objet (1) ou le dispositif de détection (20) et le dispositif d'éclairage (10) peuvent être déplacés par rapport à l'objet (1) et/ou
- le dispositif de maintien comprend une membrane (55) agencée pour recevoir l'objet (1), dans lequel la membrane sépare l'objet (1) et le milieu de transmission adéquat (53) l'un de l'autre.

2. Dispositif d'imagerie selon la revendication 1, dans lequel

- l'objet (1) peut être déplacé avec le dispositif de maintien par rapport à l'ensemble d'éléments de détection.

**3.** Dispositif d'imagerie selon la revendication 1 ou 2, dans lequel

- le dispositif de détection (20) comprend un ensemble d'éléments de détection focalisés qui sont focalisés sur un plan de détection commun dans l'objet.

**4.** Dispositif d'imagerie selon l'une des revendications précédentes, dans lequel

- les éléments de détection sont agencés le long d'un arc qui entoure au moins partiellement l'objet (1).

**5.** Dispositif d'imagerie selon l'une des revendications précédentes, dans lequel

- les éléments de détection sont agencés le long d'un arc en forme de demi-cercle ou semi-cylindrique.

**6.** Dispositif d'imagerie selon l'une des revendications précédentes, dans lequel

- les éléments de détection sont répartis le long d'une ligne incurvée et/ou le long d'une surface incurvée.

**7.** Dispositif d'imagerie selon l'une des revendications précédentes, dans lequel

- les composants optiques comprennent des miroirs optiques ou des fibres optiques situés dans la cuve (51), et/ou
- les composants optiques sont agencés pour éclairer l'objet (1) avec un motif de type en anneau.

**8.** Dispositif d'imagerie selon la revendication 7, dans lequel

- les fibres optiques comprennent un faisceau de fibres comportant plusieurs tiges qui sont agencées à des angles uniformément espacés totalisant 360°.

**9.** Dispositif d'imagerie selon l'une des revendications précédentes, dans lequel le dispositif de maintien comprend :

- un élément de maintient en forme de tige ou de plaque (52, 54) agencé pour porter l'objet (1).

**10.** Dispositif d'imagerie selon l'une des revendications précédentes, dans lequel la membrane présente au moins l'une des caractéristiques suivantes

- la membrane (55) est adaptée acoustiquement et optiquement pour éclairer l'objet (1) et détecter les signaux acoustiques,
- la membrane (55) est constituée d'au moins l'un du plastisol de polychlorure de vinyle, du polyéthylène et du latex, et
- la membrane (55) a une épaisseur inférieure à 1 mm.

**11.** Dispositif d'imagerie selon l'une des revendications précédentes, dans lequel le dispositif de maintien comprend :

- un élément de maintient en forme de bâti (56) agencé pour porter la membrane (55).

**12.** Dispositif d'imagerie selon la revendication 11, dans lequel

- l'élément de maintient en forme de bâti (56) peut être déplacé le long d'un rail de guidage (57) à l'intérieur de la cuve (51).

**13.** Procédé d'imagerie pour la formation d'une image opto-acoustique, d'un objet (1), comprenant les étapes consistant :

- à placer l'objet (1) avec un dispositif de maintien (52, 54, 55) dans une cuve (51) d'un dispositif de réception (50), un milieu de transmission adéquat (53) et un dispositif de détection (20) étant inclus dans la cuve (51),
- à éclairer l'objet (1) par l'intermédiaire des composants optiques d'un dispositif d'éclairage (10),
- à détecter des signaux acoustiques créés dans l'objet (1) par un ensemble d'éléments de détection du dispositif de détection (20), dans lequel l'objet (1) avec le dispositif de maintien (52, 54, 55) et/ou le dispositif d'éclairage (10) et/ou le dispositif de détection (20) sont déplacés les uns par rapport aux autres pendant la détection des signaux acoustiques, dans lequel le mouvement comprend une translation, et
- à reconstruire une image de l'objet (1) en analysant les signaux acoustiques,
**caractérisé en ce que**
- le dispositif d'éclairage (10) est déplacé par rapport à l'objet (1) ou le dispositif de détection (20) et le dispositif d'éclairage (10) sont déplacés par rapport à l'objet (1) et/ou
- le dispositif de maintien comprend une membrane (55) dans laquelle l'objet (1) est reçu, dans lequel la membrane sépare l'objet (1) et le milieu de transmission adéquat (53) l'un de l'autre.

**14.** Procédé d'imagerie selon la revendication 13, consistant :

- à ajuster progressivement l'objet par rapport à un plan de détection du dispositif de détection ; à éclairer l'objet avec une lumière d'éclairage pulsée ou à irradier l'objet avec une énergie électromagnétique et la détection des signaux acoustiques avec la position de l'objet ajustée, dans lequel les étapes d'éclairage ou d'irradiation et de détection sont répétées au moins deux fois avec différentes longueurs d'onde de la lumière d'éclairage ou de l'énergie électromagnétique, respectivement.

**15.** Dispositif d'imagerie (100) configuré pour la formation d'une image thermoacoustique d'un objet (1), comprenant :

- un dispositif formant générateur comprenant des composants d'irradiation agencés pour irradier l'objet (1) avec une énergie électromagnétique, en particulier dans la plage des radiofréquences,
- un dispositif de détection (20) agencé pour détecter les signaux acoustiques créés dans l'objet (1),
- un dispositif de réception (50) comprenant une cuve (51) agencée pour recevoir le dispositif de détection (20), l'objet (1) et un milieu de transmission adéquat (53), et
- un dispositif de maintien (52, 54, 55) pour positionner l'objet (1) dans le dispositif de réception (50), dans lequel
- le dispositif de détection (20) comprend un ensemble d'éléments de détection, et
- le dispositif de maintien (52, 54, 55) avec l'objet (1) et/ou le dispositif formant générateur et/ou le dispositif de détection (20) peuvent être déplacés les uns par rapport aux autres, dans lequel le mouvement comprend une translation, **caractérisé en ce que**
- le dispositif formant générateur (10) peut être déplacé par rapport à l'objet (1) ou le dispositif de détection (20) et le dispositif formant générateur (10) peuvent être déplacés par rapport à l'objet (1) et/ou
- le dispositif de maintien comprend une membrane (55) agencée pour recevoir l'objet (1), dans lequel la membrane sépare l'objet (1) et le milieu de transmission adéquat (53) l'un de l'autre.

**16.** Procédé d'imagerie pour la formation d'une image thermoacoustique d'un objet (1), comprenant les étapes

- de positionnement de l'objet (1) avec un dispositif de maintien (52, 54, 55) dans une cuve (51) d'un dispositif de réception (50), un milieu de transmission adéquat (53) et un dispositif de détection (20) étant inclus dans la cuve (51),
- d'irradiation de l'objet (1) avec une énergie électromagnétique, en particulier dans la plage des radiofréquences, par l'intermédiaire des composants d'irradiation d'un dispositif formant générateur,
- de détection des signaux acoustiques créés dans l'objet (1) par un ensemble d'éléments de détection du dispositif de détection (20), dans lequel l'objet (1) avec le dispositif de maintien (52, 54, 55) et/ou le dispositif formant générateur et/ou le dispositif de détection (20) sont déplacés les uns par rapport aux autres pendant la détection des signaux acoustiques, dans lequel le mouvement comprend une translation, et
- de reconstruction d'une image de l'objet (1) en analysant les signaux acoustiques, **caractérisé en ce que**
- le dispositif formant générateur (10) est déplacé par rapport à l'objet (1) ou le dispositif de détection (20) et le dispositif formant générateur (10) sont déplacés par rapport à l'objet (1) et/ou
- le dispositif de maintien comprend une membrane (55) dans laquelle l'objet (1) est reçu, dans lequel la membrane sépare l'objet (1) et le milieu de transmission adéquat (53) l'un de l'autre.

FIG. 1

FIG. 2

FIG. 3

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6567688 B
- WO 2010009747 A
- WO 2011000389 A

**Non-patent literature cited in the description**

- **D. RAZANSKY ; V. NTZIACHRISTOS.** *Med. Phys.,* 2007, vol. 34, 4293-4301
- **K. V. LARIN et al.** *Journal of Physics D-Applied Physics,* 2005, vol. 38 (15), 2645-2653
- American National Standards for the Safe Use of Lasers. ANSI Z136.1. American Laser Institute, 2000